# DEMANDE DE BREVET EUROPEEN

(11) **EP 3 045 908 A1**
(43) Date de publication de la demande: **20.07.2016**
(21) Numéro de dépôt: 16150893.2
(22) Date de dépôt: 12.01.2016
(51) Int. Cl.: G01N 31/22, G01N 33/22, G01N 21/78

(54) **ANALYSE DU PHÉNOL EN PHASE LIQUIDE**

(30) Priorité: 13.01.2015 FR 1550243
(71) Demandeur: COMMISSARIAT A L'ENERGIE ATOMIQUE ET AUX ENERGIES ALTERNATIVES, 75015 Paris (FR); CENTRE NATIONAL DE LA RECHERCHE SCIENTIFIQUE -CNRS-, 75016 Paris (FR)
(72) Inventeur: TRAN-THI, Thu-Hoa, 92120 Montrouge (FR); MUGHERLI, Laurent, 91640 Vaugrigneuse (FR); BORTA, Ana, 91120 Palaiseau (FR)
(74) Mandataire: Cabinet Plasseraud

(57) **Abrégé**

Composition liquide réactive de détection du phénol ou de composés du phénol comprenant un réactif capable de générer un produit coloré par formation d'une liaison avec le phénol, un composé oxydant ou mélange de composés oxydants, un composé basique ou mélange de composés basiques, en ce que le ratio [réactif coloré] / [composé oxydant] est de 1/2 à 50/1, ayant un pH supérieur à 7, kit de mise en oeuvre de la composition et procédé en phase liquide d'analyse d'un fluide pouvant contenir du phénol ou un composé du phénol.

## Description

La présente invention concerne une composition réactive et un procédé d'analyse du phénol ou des composés du phénol en phase liquide et leurs applications.

Le phénol et ses composés sont des composés hautement toxiques. Ceux-ci sont largement utilisés dans la production ou la fabrication d'une grande variété des composés aromatiques y compris les explosifs, les engrais, le gaz d'éclairage, les peintures, le caoutchouc, les textiles, les produits pharmaceutiques, les parfums et les matières plastiques, comme par exemple les polycarbonates ou les résines époxy. Le phénol est couramment utilisé comme intermédiaire dans les procédés chimiques industriels de synthèse et notamment dans les industries du pétrole, des matières plastiques, des colorants, du cuir, du papier, du savon. Dans une moindre mesure, le phénol et ses composés entrent dans la composition des cosmétiques et des médicaments. Dans certains pays, le phénol est également utilisé comme répulsif contre les moustiques et comme insecticide et herbicide pour l'agriculture. Par conséquent, le phénol et ses composés se retrouvent dans l'environnement majoritairement par des rejets industriels, mais aussi par l'activité domestique (métabolisme humain et animal, eaux usées, combustion du fioul domestique). Des traces de phénol sont aussi présentes dans les gaz d'échappement des automobiles ou la fumée de tabac. Depuis 2006 l'utilisation du phénol comme désinfectant a été interdite en France. Cependant des composés du phénol, comme par exemple les chlorophénols sont souvent utilisés en substitution. Ainsi le phénol et ses composés sont omniprésents, dans l'air et dans l'eau, à des concentrations variables en fonction de l'activité humaine.

Le phénol est toxique par inhalation, par contact cutané et par ingestion. Il est également susceptible d'induire des anomalies génétiques. En cas de contact, il provoque des brulures de la peau et des lésions oculaires. Des expositions répétées ou prolongées peuvent induire des effets graves pour les organes, tels que : perturbations digestives, maux de tête, salivation, anorexie, vomissements et perte d'appétit. Il provoque également des dommages de la moelle osseuse.

Les valeurs limites d'exposition professionnelle contraignantes dans l'air des locaux de travail ont été établies en France et en Union Européenne à 2 ppm (7.8 mg/m³) pour une moyenne pondérée sur 8 heures et à 4 ppm (15.6 mg/m³) pour une exposition court terme de 15 min maximum (selon fiche toxicologique de l'Institut National de Recherche et de Sécurité - INRS).

A ce jour, la détection sensible, sélective et fiable du phénol dans un mélange gazeux s'effectue par une méthode indirecte avec prélèvement de gaz puis analyse par la suite en laboratoire.

Pour détecter le phénol en solution, il faut utiliser une méthode différente, la référence étant la méthode colorimétrique fondée sur la 4-aminoantipyrine (AAP), dans laquelle le phénol ne peut pas être ajouté en dernier, et les réactifs, préalablement conservés séparément, doivent être ajoutés au dernier moment, ce qui rend la méthode très complexe. Enfin ces méthodes ne peuvent être utilisées que pour la détection, et en aucun cas à la dépollution, si la présence de phénol est établie.

Il existe de nombreuses méthodes de détection du phénol en solution aqueuse ou dans l'air.

Les méthodes de détection se décomposent principalement en trois familles : les méthodes chromatographiques, les méthodes électrochimiques et les méthodes colorimétriques. D'autres méthodes existent également et sont en cours de développement comme par exemple les méthodes basées sur une micro-variation de masse (résonateurs piézoélectriques).

Les méthodes chromatographiques nécessitent des appareillages coûteux et des procédures complexes. Elles induisent en plus un délai de réponse quand elles sont utilisées de manière indirecte (prélèvement sur le terrain puis analyse dans le laboratoire).

Les méthodes électrochimiques ne sont pas aisément adaptables pour la détection dans l'air et nécessitent souvent l'ajout d'électrodes modifiées contenant des nanoparticules, polymères ou enzymes. Les coûts de telles électrodes et leur vieillissement dans des conditions d'utilisation réelles peuvent constituer un frein à leur industrialisation.

Les méthodes chromatographiques et électrochimiques présentent une complexité intrinsèque soit dans la matière première (enzymes, nanoparticules...), soit dans la préparation (matériaux) soit dans leur protocole de mise en oeuvre (prélèvement, extraction, concentration, purification, ajout des autres réactifs, dosage), soit dans la mesure proprement dite (appareillages sophistiqués, encombrants). A cette complexité est ainsi associé un coût assez important et un temps d'obtention des résultats qui peut être long. De plus, une certaine expertise est requise pour passer les échantillons et interpréter les résultats, car ces méthodes sont loin d'être simples d'emploi.

Les méthodes colorimétriques ont l'avantage d'être plus simples d'utilisation en termes de préparation, prélèvement, mesure, et obtention des résultats. Ces méthodes sont en général directes (pas d'analyse *a posteriori* en laboratoire). La réponse est donc plus rapide. De plus l'appareillage nécessaire est peu couteux, et plus compact.

Dans la littérature, il existe un grand nombre de méthodes de détection colorimétriques du phénol ou de ses composés en solution, utilisant des réactifs en milieu acide, tels que l'acide p-diazobenzènesulfonique (DABS) qui conduit à une couleur jaune, le mélange Fe(II)/1,10-phénantroline qui produit une coloration rose, le mélange formaldéhyde-acide sulfurique (FSA) et sa coloration pourpre, ou encore le bromure d'iode qui forme un produit absorbant dans l'UV. Des réactions en milieu basique ont aussi été décrites, utilisant par exemple le réactif de Folin-Ciocalteu, qui développe une coloration bleue. D'autres réactifs nécessitent l'extraction par un solvant organique du produit coloré, comme par exemple le produit marron du réactif Millon, le produit rose du MBTH (3-Methyl-2-benzothiazolinone hydrazone hydrochloride monohydrate) en présence d'un oxydant, ou encore les complexes de transfert de charge bleus issus de la 2,3-dichloro-5,6-dicyano-p-benzoquinone (DDQ). Certains de ces réactifs sont coûteux, d'autres toxiques. De plus ils peuvent nécessiter une étape d'extraction avant analyse de la couleur. Ils ne sont pas tous suffisamment sensibles et sélectifs.

La méthode le plus couramment employée qui confère une sensibilité et une sélectivité satisfaisante, probablement car c'est la plus simple, est fondée sur l'utilisation de 4-aminoantipyrine (AAP). C'est d'ailleurs la méthode de référence citée par l'Institut National de l'Environnement Industriel et des Risques (INERIS). La méthode implique la formation d'un complexe quinoïque entre la 4-aminoantipyrine et le phénol en milieu basique (préférentiellement pH =9 ou 10) et en présence de l'oxydant hexacyanoferrate de potassium. La coloration se développe après 5 min pour des concentrations supérieures à 1 mg/L soit environ 10 µM (193 ppb) et le composé quinoïque présente un maximum d'absorption vers 510 nm. Il est à noter que pour des concentrations inférieures à 1 mg/L, l'INERIS recommande trois extractions successives au chloroforme, ce qui rend l'opération notablement plus complexe. Dans la littérature, cette réaction est décrite avec différents composés de l'AAP et différents oxydants, ainsi qu'avec différents rapports [AAP]/[oxydant], par exemple Analytical Chimica Acta, 467, 2002, 105-114, Jour. Chem. Soc. Pak., 27, 2005, 271-274, ou Jour. Organ. Chem., 8, 1943, 417-428.

Les composés d'AAP abordés dans certaines publications sont intéressants, notamment ceux qui permettent d'augmenter la sensibilité grâce à un coefficient d'extinction molaire plus élevé. Cependant on ne peut pas se les procurer aisément car ils sont synthétisés à façon pour la plupart. La majorité des méthodes fondées sur l'AAP décrites dans la littérature permettent une détection sélective et sensible de l'ordre de quelques ppb, mais elles utilisent toutes une étape d'extraction au chloroforme qui complexifie le protocole et allonge sa durée. Si l'on veut s'affranchir de cette étape d'extraction, les recommandations de l'INRS et les rares publications avec détection directe en solution aqueuse indiquent une baisse significative de la sensibilité : la méthode ne fonctionne alors correctement que pour des concentrations supérieures à 200 ppb (1 mg/L, 10 µM). Toutes ces méthodes utilisent un excès d'oxydant, et l'ordre de mise en oeuvre des réactifs est toujours le même, à savoir: 1) solution tampon, 2) phénol, 3) AAP, 4) oxydant. Le temps d'attente avant de mesurer l'absorbance varie de 5 à 30 min.

Les tentatives pour adapter cette méthode de référence en phase liquide à la détection du phénol en phase gazeuse ne sont pas convaincantes. En effet, seulement deux brevets ont été déposés, et l'adaptation a nécessité une importante complexification du procédé [Brevets CN102590198 et CN202362242U]. Plusieurs étapes sont ajoutées, notamment dissolution en milieu acide, distillation, stripage, chauffage, extraction. De plus, dans un des brevets, les réactifs sont ajoutés de manière séquentielle, ce qui complexifie encore le procédé [Brevet CN202362242U].

L'exposé qui précède montre qu'il n'existe pas à l'heure actuelle de méthode colorimétrique simple et directe fondée sur l'utilisation d'AAP ou de ses composés, qui soit sélective et sensible, et qui permette la détection du phénol en solution en l'ajoutant en dernier, en d'autres termes il n'existe pas de procédé où l'addition de l'échantillon constitue la dernière étape.

Les méthodes développées pour la détection du phénol dans l'air sont bien moins nombreuses. A une écrasante majorité, les méthodes connues sont effectuées en plusieurs étapes. Premièrement, le prélèvement de l'air contenant du phénol est effectué au travers d'un tube rempli de gel de silice ou de résine (typiquement XAD7), puis ce prélèvement est suivi d'une désorption par des solvants et d'une dérivation par un agent de silylation, ensuite, un dosage est effectué, par chromatographie en phase gazeuse avec détection par ionisation de flamme ou par chromatographie en phase liquide avec détection UV ou électrochimique.

La seule méthode directe, c'est-à-dire sans analyse *a posteriori* en laboratoire, consiste à employer des tubes gradués GASTEC (n °60) ou DRAEGER (Phénol 1/b, MSA Phénol-1). Le principe de la détection est fondé sur la réaction du phénol en milieu acide avec (NH₄)₂Ce(NO₃)₆ (pour GASTEC) et Ce(SO₄)₂ (pour DRAEGER), qui donne un changement de couleur de jaune à brun-vert. Le produit coloré se propage dans le tube avec une vitesse dépendant de la concentration en phénol pour un flux gazeux pompé à un débit déterminé par le fabricant. La lecture de la concentration se fait grâce aux graduations imprimées sur le tube. Le plus performant est le tube GASTEC, avec une gamme de détection de 0,4 à 187 ppm avec une erreur de 10% à 15% indiquée par le fabricant. L'avantage de cette détection est sa simplicité, mais la réaction de détection n'est pas sélective, et il faut appliquer un facteur de correction en fonction de l'humidité et de la température. Ainsi, ces tubes n'assurent ni la sélectivité ni la précision nécessaires à une comparaison à une valeur limite d'exposition professionnelle selon l'INRS. Ces tubes ne peuvent pas être utilisés dans l'eau, ce qui constitue une limitation supplémentaire au procédé.

Il n'existe donc à l'heure actuelle pas de méthode colorimétrique directe, sélective et sensible qui permette la détection du phénol ou des composés du phénol en phase liquide. De plus, aucune méthode universelle permettant la détection à la fois dans un fluide tel un mélange gazeux comme l'air ou dans une solution, par exemple aqueuse, par simple mise en contact d'une composition prête à l'emploi avec le fluide, n'a été mise au point jusqu'à présent.

Il serait donc souhaitable de disposer d'une telle méthode. La méthode devrait permettre une détection directe, optique ou visuelle réalisable sur le terrain avec un matériel portatif, pour éviter le transport des échantillons à tester dans un laboratoire équipé pour réaliser une telle mesure. La méthode devrait aussi être simple. Elle devrait pouvoir être utilisable aussi bien pour la détection du phénol dans un mélange gazeux ou liquide. La méthode devrait pouvoir si possible non seulement être utilisée pour la détection, mais aussi pour la dépollution sélective du phénol et de ses composés.

La présente invention permet de résoudre tout ou partie des problèmes techniques liés à la détection du phénol évoqués ci-dessus.

Après de longues recherches, les inventeurs ont mis au point une méthode qui s'inspire de la réaction colorimétrique en solution fondée sur l'AAP, mais avec des changements majeurs, qui ont conduit à une méthode précise et fiable mais considérablement simplifiée.

Avec la méthode de référence, l'oxydant est toujours utilisé en excès et l'ordre d'ajout des réactifs est très important : 1) solution tampon, 2) phénol, 3) AAP, 4) oxydant. En présence d'un excès d'oxydant, si cet ordre n'est pas respecté, le changement de couleur ne se produit pas. Pour une utilisation simple, la demanderesse a imaginé de placer tous les réactifs pré-mélangés en présence de phénol et ne pas avoir d'autre opération à faire par la suite, à part lire le résultat de la mesure. Ainsi, le phénol (ou composé du phénol) serait forcément mis au contact des autres réactifs en dernier.

La présente invention est fondée sur un mélange de réactifs pré-mélangé grâce à 2 innovations : la modification de l'ordre d'ajout des réactifs et la modification du ratio [AAP]/[hexacyanoferrate de potassium]. Le pré-mélange de réactifs permet de détecter directement le phénol par mise en contact du fluide pouvant contenir du phénol avec une composition réactive de l'invention, par exemple par simple barbotage dans le cas d'un fluide gazeux.

C'est pourquoi la présente demande a pour objet une composition liquide réactive de détection du phénol ou de composés du phénol ou de composés du phénol caractérisée en ce qu'elle comprend
- un réactif capable de générer un produit coloré par formation d'une liaison avec le phénol,
- un composé oxydant ou mélange de composés oxydants,
- un composé basique ou mélange de composés basiques,
en ce que le ratio [réactif capable de générer un produit coloré] / [composé oxydant] est de 1/2 à 50/1,
et en ce qu'elle a un pH supérieur à 7.

Dans le cadre de la présente invention, le réactif capable de générer un produit coloré est un composé qui permet à une couleur de se développer par formation d'une liaison habituellement covalente avec le phénol ou un composé du phénol, pour former un produit coloré, par exemple un composé quinoïque. Le réactif capable de générer un produit coloré est de préférence l'AAP mais peut-être également un dérivé de l'AAP, comme par exemple un composé correspondant à la formule I 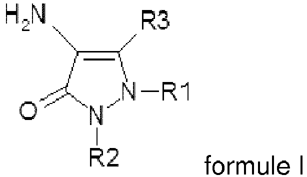 où : R1 est un radical alkyl renfermant de 1 à 30, notamment 1 à 12, préférentiellement 1 à 6 atomes de carbone, linéaire ou ramifié, ou un radical aryl, de préférence phényl, préférentiellement un radical C₆H₅, CH₃ ou C₂H₅ ; R2 est un radical phényl ou para-aminophényl, préférentiellement ce dernier ; R3 est un radical alkyle renfermant de 1 à 30, notamment 1 à 12, préférentiellement 1 à 6 atomes de carbone, linéaire ou ramifié, ou phényl éventuellement substitué, de préférence en position para, par exemple par un radical amino ou halogèno, tel un radical para-aminophényl ou parachlorophényl. Le réactif capable de générer un produit coloré est de préférence l'AAP.

La composition liquide réactive de l'invention contient également un composé oxydant ou mélange de composés oxydants. Le composé oxydant ou les composés oxydants sont avantageusement choisis dans le groupe constitué par le persulfate de potassium, le peroxomonosulfate de potassium ; le peroxyde d'hydrogène ou l'hexacyanoferrate de potassium. On utilise avantageusement ce dernier qui permet un bon compromis entre douceur de l'oxydation et vitesse de réaction. D'autres oxydants que ceux de la liste non exhaustive ci-dessus sont possibles.

La composition liquide réactive de l'invention contient également un composé basique ou mélange de composés basiques. Étant donné que la composition réactive de l'invention est liquide, on utilise avantageusement une solution tampon, comme par exemple un tampon phosphate, 2-amino-2-(hydroxyméthyl)-1,3-propanediol, carbonate, glycine-soude ou borate, préférentiellement un tampon borate, pour obtenir un pH basique égal à de préférence environ 10. Le composé basique ou mélange de composés basiques est nécessaire pour le bon déroulement de la réaction colorée, tout en apportant en dernier le phénol ou composé du phénol.

A noter que dans la présente demande, classiquement l'article indéfini "un" doit être considéré comme un pluriel générique (signification de "au moins un" ou encore "un ou plusieurs"), sauf lorsque le contexte montre le contraire (1 ou "un seul"). Ainsi, par exemple, lorsque l'on dit ci-dessus que la composition liquide réactive de l'invention comprend un réactif capable de générer un produit coloré ou comprend un composé basique, il convient de comprendre que la composition liquide réactive contient un ou plusieurs réactifs capable de générer un produit coloré ou composés basiques.

Dans des conditions préférentielles de mise en oeuvre de l'invention, le réactif capable de générer un produit coloré est l'AAP, et le composé basique ou mélange de composés basiques est choisi parmi un tampon phosphate ou un tampon borate, préférentiellement un tampon borate, et le composé oxydant ou les composés oxydants est l'hexacyanoferrate de potassium ou un mélange d'hexacyanoferrate de potassium et un ou plusieurs autres composés oxydants.

Un excès de réactif capable de générer un produit coloré, typiquement supérieur à 50/1, n'est pas souhaitable car la stabilité de la coloration est réduite, et le pic du réactif capable de générer un produit coloré peut interférer avec celui du produit coloré issu de la réaction colorée.

Le ratio [réactif capable de générer un produit coloré] / [composé oxydant] est de préférence de 1/2 à 25/1, avantageusement de 1/2 à 20/1, notamment de 1/2 à 10/1, particulièrement de 1/2 à 5/1, et tout particulièrement de 1/1.

Dans la composition liquide réactive de l'invention, les réactifs peuvent être présents en quantités telles que, après mélange, les concentrations en réactif capable de générer un produit coloré soient égales à 1,0.10⁻¹ M à 5,0.10⁻³ M, avantageusement de 1,0.10⁻² M à 5,0.10⁻⁴ M, de préférence de 1,0.10⁻³ M à 5.10⁻⁵ M , notamment de 2,0.10⁻⁴ M à 1,0.10⁻⁵ M, tout particulièrement de 6,0.10⁻⁴ M à 4,0.10⁻⁵ M.

Dans la composition liquide réactive de l'invention, les réactifs peuvent être présents en quantités telles que, après mélange, les concentrations en oxydant soient égales à: 2,0.10⁻¹ M à 2,0.10⁻⁴ M, avantageusement de 2,0.10⁻² M à 2,0.10⁻⁵ M, de préférence de 2,0.10⁻³ M à 2.10⁻⁶ M , notamment de 4,0.10⁻⁴ M à 4,0.10⁻⁷ M , tout particulièrement de 1,2.10⁻⁴ M à 8,0.10⁻⁵ M.

De préférence la composition réactive est essentiellement constituée des composants ci-dessus, et tout particulièrement constituée desdits composants.

Si dans le cadre de l'invention on peut utiliser une composition réactive et liquide prête à l'emploi, on peut aussi procurer les différents constituants séparément, pour préparer extemporanément le mélange qui pourra être utilisé pour la détection ou le dosage du phénol ou un de ses composés.

C'est pourquoi la présente demande a aussi pour objet un kit caractérisé en ce qu'il comprend séparément
- un réactif capable de générer un produit coloré,
- un composé oxydant ou mélange de composés oxydants,
- un composé basique ou un mélange de composés basiques,
où le ratio [réactif capable de générer un produit coloré] / [composé oxydant] est de 1/2 à 50/1.

S'ils peuvent être apportés en solution, le réactif capable de générer un produit coloré, le composé basique ou mélange de composés basiques, et le composé oxydant sont de préférence sous forme solide.

Ainsi, il suffit de mettre un des constituants en solution, de préférence le composé basique ou mélange de composés basiques, puis de mélanger l'ensemble des constituants.

Dans des conditions préférentielles de mise en oeuvre du kit, le réactif capable de générer un produit coloré et le composé oxydant sont sous forme solide, et le composé basique ou mélange de composés basiques se trouve en solution. Le composé basique ou mélange de composés basiques est préférentiellement du tampon borate à pH 9.

L'invention pourra en particulier prendre la forme d'un kit de détection comprenant une solution basique et un récipient, préférentiellement protégé de la lumière. Ce récipient contient, dans des emplacements séparés, avantageusement sous atmosphère inerte, le réactif capable de générer un produit coloré et le composé oxydant sous forme solide.

Le récipient est de préférence un récipient clos pourvu de zones (fenêtres optiques) permettant la détection optique par un appareillage approprié, ainsi que d'ouvertures pour la circulation ou l'ajout de liquides ou de gaz.

Ainsi, on peut ajouter la solution basique aux constituants solides et mélanger l'ensemble des réactifs, pour obtenir une solution prête à l'emploi.

Dans un mode particulier de mise en oeuvre de du kit, le récipient est muni d'une séparation amovible procurant deux compartiments juxtaposés dans lesquels sont installés dans l'un, le réactif capable de générer un produit coloré et dans l'autre le composé oxydant, sous forme solide, ce mélange pouvant être réalisé en retirant la séparation amovible.

La détection de phénol ou d'un composé du phénol dans un fluide liquide peut se faire par exemple directement via les ouvertures du récipient. Le barbotage d'un mélange gazeux contenant du phénol ou un composé du phénol est possible directement dans le récipient.

La détection peut se faire directement en reliant un système de détection optique aux fenêtres optiques du récipient.

Dans d'autres conditions préférentielles de mise en oeuvre des kits de l'invention, un récipient comprend trois compartiments, les 2 premiers étant analogues à ceux ci-dessus, et le troisième compartiment étant réservé au composé basique ou mélange de composés basiques sous forme solide, par exemple un mélange de borax et de soude pour reconstituer un tampon borate pH 9.

On peut effectivement préparer le tampon ajuste au bon pH puis le lyophiliser, mais la quantité de solide correspondant au tampon dans le kit serait alors très faible de (l'ordre du mg) et donc difficile à peser. Le tampon se conservant bien en solution ; le rapport bénéfice/complication ne serait selon nous pas intéressant. Mais c'est possible.

Pour procéder à une détection ou un dosage, il suffit d'ajouter un solvant comme l'eau puis de procéder au mélange. Un tel kit à une durée de vie particulièrement longue.

Les modes de réalisation ci-dessus procurent des moyens prêts à l'emploi dans des appareils de mesures spectrophotométriques. Mais l'homme de l'art comprend qu'il n'est pas nécessaire que le récipient dispose de fenêtres optiques, et qu'il est donc possible, une fois le mélange réalisé, de le transférer dans une cuve adaptée à des mesures spectrophotométriques.

La composition réactive de l'invention peut être facilement préparée par simple mélange des différents constituants, qui eux-mêmes sont facilement disponibles et bon marché.

La présente demande a aussi pour objet un procédé de préparation d'une composition réactive ci-dessus, caractérisé en ce que l'on mélange les différents constituants selon des méthodes connues en elle-même.

La présente demande a également pour objet un procédé de préparation d'un kit ci-dessus, caractérisé en ce que l'on procure les différents constituants de la composition réactive, par exemple dans des sachets, dans des flacons, ou directement dans un récipient de préférence muni d'une ou plusieurs cloison de séparation pour éviter le mélange les différents constituants avant utilisation.

Les compositions réactives et kits objet de la présente invention possèdent de très intéressantes propriétés. Dans les compositions réactives et kits ci-dessus, on obtient une solution prête à l'emploi. Si du phénol ou un composé du phénol est présent ou suspecté dans un liquide, il est suffisant d'ajouter un certain volume du liquide au système. S'il est présent, le phénol ou composé du phénol va alors réagir avec les réactifs présents dans le récipient pour former un composé coloré présentant un maximum d'absorption. Cette propriété d'absorbance de la solution peut être reliée à la concentration en phénol ou composé du phénol grâce à une courbe de calibration. A l'absorbance est associé un changement de couleur de la solution visible à l'oeil nu. La détection peut donc être qualitative ou quantitative. Pour un gaz ou mélange de gaz, il suffit de faire barboter le fluide gazeux dans la solution prête à l'emploi ou préparée extemporanément.

Ces propriétés sont illustrées ci-après dans la partie expérimentale. Elles justifient l'utilisation des compositions réactives et kits ci-dessus décrits, dans l'analyse d'un fluide pouvant contenir du phénol ou un composé du phénol.

C'est pourquoi la présente demande a aussi pour objet un procédé en phase liquide d'analyse d'un fluide pouvant contenir du phénol ou un composé du phénol caractérisé en ce que l'on met en contact le fluide avec une composition liquide réactive de détection du phénol ou de composés du phénol définie ci-dessus et en ce que l'on observe ou mesure l'éventuelle coloration obtenue.

Les composés du phénol sont par exemple des alkoxyphénols, des nitrophénols, des chlorophénols, ou des phénols polyaromatiques. On peut citer particulièrement le 4-méthoxyphénol, le 2-nitrophénol, le 2,4-dichlorophénol, ou le naphtol, mais encore le crésol, le catéchol, le résorcinol, 2,4-diméthylphénol, 4-chloro-3-méthylphénol, le 4-nitrophénol, le 2,4-dinitrophénol, le 4,6-dinitrophénol, le 4-chlorophénol, le 2-chlorophénol, le 2,4,6-trichlorophénol, le pentachlorophénol .

Dans des conditions de mise en oeuvre du procédé ci-dessus, le fluide pouvant contenir du phénol ou un composé du phénol est un gaz ou un mélange de gaz. Dans d'autres conditions de mise en oeuvre du procédé ci-dessus, le fluide est un liquide.

Lorsque le fluide est un liquide, ce liquide est par exemple l'eau, l'éthanol, le THF ou le DMSO, préférentiellement en mélange avec de l'eau.

Lorsque le fluide est un gaz ou mélange de gaz, ce fluide est par exemple l'air, des gaz vecteur neutres comme l'azote ou l'argon.

Ces fluides peuvent provenir notamment d'industrie comme par exemple l'industrie du pétrole, des plastiques, peintures, colorants.

Dans des conditions préférentielles de mise en oeuvre d'un kit de l'invention, le procédé ci-dessus décrit comprend une étape consistant à procurer un récipient clos pourvu de zones (fenêtres optiques) permettant la détection optique par un appareillage approprié, ainsi que d'ouvertures pour la circulation ou l'ajout de liquides ou de gaz, ledit récipient contenant, dans des compartiments séparés, un réactif capable de générer un produit coloré et un composé oxydant sous forme solide.

Ainsi, on peut ajouter la solution basique aux constituants solides et mélanger l'ensemble des réactifs, pour obtenir une solution prête à l'emploi.

Dans un mode particulier de mise en oeuvre du kit, le récipient est muni d'une séparation amovible procurant deux compartiments juxtaposés dans lesquels sont installés dans l'un, le réactif capable de générer un produit coloré et dans l'autre le composé oxydant, sous forme solide, , on retire la séparation amovible, puis on ajoute une solution basique pour obtenir la composition liquide réactive de détection du phénol ou de composés du phénol de l'invention.

Si pour la mise en oeuvre du procédé ci-dessus on utilise une grande quantité de composition réactive, le procédé peut être utilisé pour la dépollution sélective du phénol et de ses composés, en particulier lorsque le phénol ou ses composés se trouvent dans un fluide gazeux.

C'est pourquoi la présente demande a tout autant pour objet un procédé pour la dépollution sélective du phénol ou de ses composés, caractérisé en ce que l'on met en contact un fluide contenant du phénol ou un de ses composés avec une composition réactive définie ci-dessus. Le fluide et en particulier un fluide gazeux.

Les conditions préférentielles de mise en oeuvre des compositions liquides réactives ci-dessus décrites, choix des réactifs préférentiels, proportions etc. s'appliquent également aux autres objets de l'invention visés ci-dessus, notamment aux procédés ou kits.

Les exemples qui suivent illustrent la présente demande et l'invention sera mieux comprise si l'on se réfère aux dessins annexés sur lesquels
- la figure 1 (figure 3) est une courbe de suivi par spectroscopie d'absorption UV/Visible de la formation d'un produit coloré par barbotage de phénol contenu dans un liquide dans une composition réactive de la présente invention;
- la figure 2 (figure 4) représente une courbe de suivi de l'évolution de l'absorbance dans le temps à 506 nm due à la formation de produit coloré par barbotage de phénol contenu dans un liquide dans une composition réactive de la présente invention au cours du temps;
- la figure 3 (figure 6) est une courbe de suivi par spectroscopie d'absorption UV/Visible de la formation d'un produit coloré par barbotage de phénol contenu dans un gaz dans une composition réactive de la présente invention;
- la figure 4 (figure 7) représente une courbe de suivi de l'évolution de l'absorbance dans le temps à 506 nm due à la formation de produit coloré par barbotage de phénol contenu dans un gaz dans une composition réactive de la présente invention au cours du temps;
- la figure 5 (figure 8) représente une courbe de calibration pour la détection de phénol dans un liquide;
- la figure 6 représente une courbe de calibration pour la détection de phénol dans un gaz.
- la figure 7 représente un mode de réalisation d'un kit de l'invention.

### Exemple 1 : Composition réactive de détection du phénol ou de composés du phénol

2,4 mL d'une solution de tampon borate 0,016 M à pH 9 sont ajoutés dans une cuve de pré-mélange où les réactifs sont présents en quantités telles que, après mélange, les concentrations en réactif capable de générer un produit coloré AAP et oxydant hexacyanoferrate de potassium soient toutes les deux égales à 8,0.10⁻⁴ M.

On obtient ainsi une composition réactive liquide prête à l'emploi utilisable pour le dosage du phénol ou de composés du phénol. Cette composition réactive peut produire en présence de phénol ou de composé du phénol un composé quinoïque coloré présentant pour le phénol un maximum d'absorption proche de 510 nm. La composition a un pH égal à 9.

### Exemple 2 : Composition réactive de détection du phénol ou de composés du phénol

2,4 mL d'une solution de tampon borate 0,016 M à pH 9 sont ajoutés dans une cuve où les réactifs sont présents en quantités telles que, après mélange, les concentrations en AAP et hexacyanoferrate de potassium soient toutes les deux respectivement égales à 1,6 10⁻² M et 8,0.10⁻⁴ M.

On obtient ainsi une composition réactive liquide prête à l'emploi utilisable pour le dosage du phénol ou de composés du phénol. Cette composition réactive peut produire en présence de phénol ou de composé du phénol un composé quinoïque coloré présentant pour le phénol un maximum d'absorption proche de 510 nm. La composition a un pH égal à 9.

### Exemple 3 : Détection du phénol ou de composés du phénol dans un mélange liquide

0,6 mL de phénol 10⁻⁴ M sont ajoutés dans une cuve contenant le réactif prêt à l'emploi de détection du phénol ou de composés du phénol de l'exemple 1.

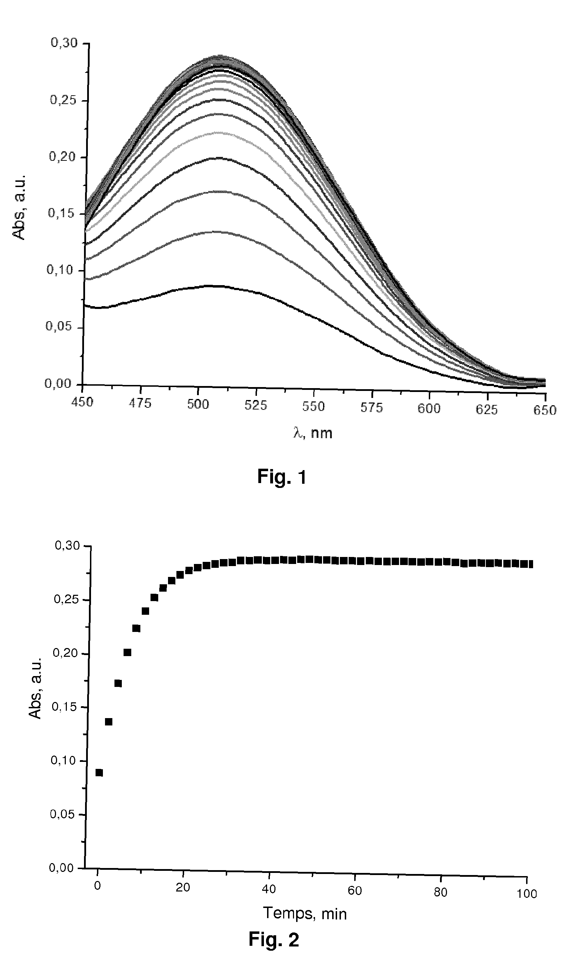

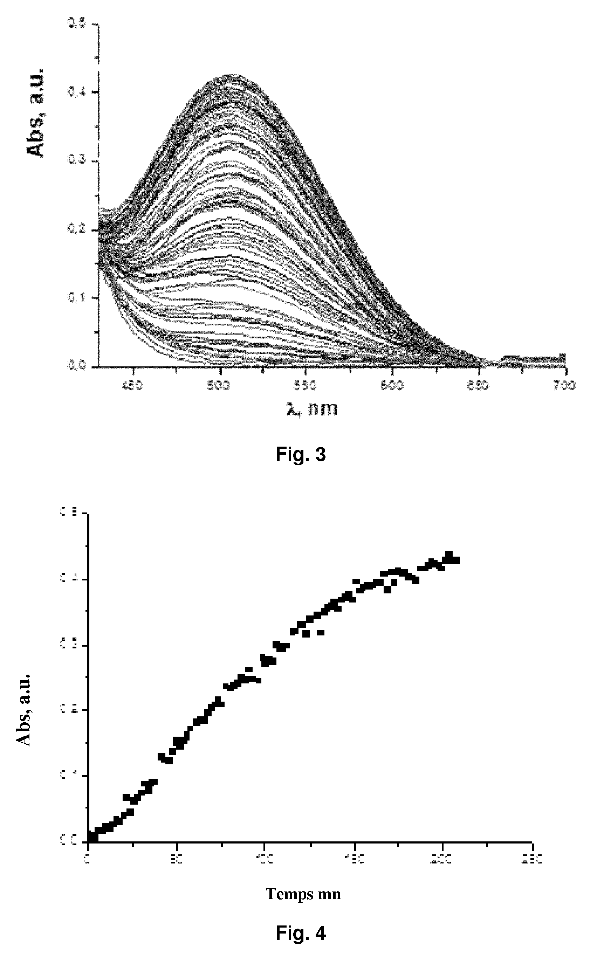

Le suivi par spectroscopie d'absorption UV-Visible au cours du temps permet d'observer l'apparition d'un pic à 506 nm, caractéristique du produit de réaction entre le réactif capable de générer un produit coloré et le phénol en présence de l'oxydant. L'intensité de ce pic augmente au cours de l'exposition puis reste stable, ce qui signifie que le phénol est progressivement consommé par la réaction, puis que la quantité du produit coloré quinoïque résultant de la réaction reste stable. La courbe correspondante est montrée sur la figure 1 (figure 3 d'origine). La stabilité de la coloration obtenue s'observe également par le plateau de la figure 2 (4 d'origine). Ainsi, la détection du phénol dans un mélange liquide est possible, et peut de plus être qualitative ou quantitative.

### Exemple 4 : Détection du phénol dans un mélange gazeux

On a fait barboter un flux gazeux continu à 100 mL/min contenant une concentration fixe de 950 ppb de phénol dans une cuve contenant la composition réactive prête à l'emploi de détection du phénol ou de composé du phénol de l'exemple 2. Le flux de gaz est apporté directement dans la cuve de mesure par un tube de quelques mm de diamètre au bout duquel est adaptée une aiguille de seringue qui est maintenue dans le liquide. L'aiguille est placée de manière à ce que les bulles ne passent pas dans le chemin optique lors de la mesure.

Une source deutérium-halogène (Ocean Optics, DH-2000-BAL) éclaire la cuve et des spectres d'absorption sont enregistrés par un spectrophotomètre UV-Visible (Ocean Optics, QE65000). Le suivi de l'absorbance en fonction du temps permet d'observer l'apparition d'un pic à 506 nm, caractéristique du produit de réaction entre le réactif capable de générer un produit coloré AAP et le phénol en présence de l'oxydant. L'intensité de ce pic augmente au cours de l'exposition puis commence à se stabiliser, ce qui signifie que la molécule sonde est progressivement consommée par la réaction. Les résultats obtenus sont montrés respectivement sur les figures 3 et 4.

Cette propriété d'absorbance de la solution peut être reliée à la concentration en phénol grâce à une courbe de calibration. A l'absorbance est associé un changement de couleur de la solution visible à l'oeil nu. Ainsi, la détection du phénol dans un mélange gazeux est possible, et peut de plus être qualitative ou quantitative.

### Expérimentation 1 : Courbe de calibration du phénol dans un mélange liquide

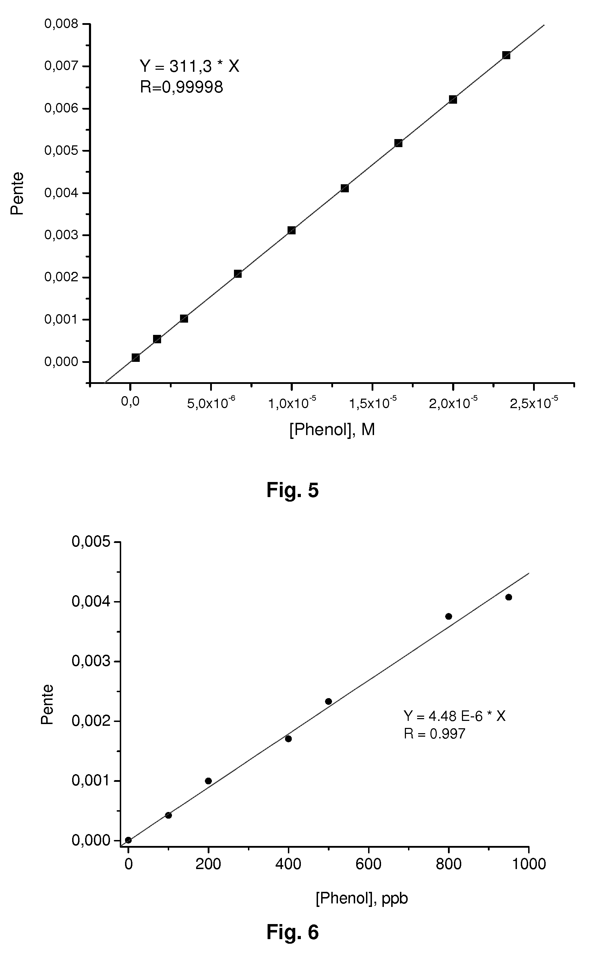

Le même procédé qu'à l'exemple 3 a été répété pour différentes concentrations de phénol. La vitesse de formation du produit coloré de réaction entre le phénol et les réactifs de la composition réactive de l'exemple 1 est dépendante de la concentration de phénol en solution. Ainsi, le tracé de la valeur des pentes en fonction de la concentration donne la courbe de calibration pour la détection du phénol. La courbe de calibration est reproduite sur la figure 5.

En conséquence, la pente obtenue est liée à la concentration de phénol et il est possible grâce à cette courbe de déterminer la concentration d'un mélange contenant une quantité de phénol inconnue.

### Expérimentation 2: Courbe de calibration du phénol dans un mélange gazeux

Le même procédé qu'à l'exemple 4 est répété pour différentes concentrations de phénol. La vitesse de formation du produit de réaction entre le phénol et les réactifs est dépendante de la concentration de phénol en solution. Ainsi, le tracé de la valeur des pentes en fonction de la concentration donne la courbe de calibration pour la détection du phénol (figure 6).

En conséquence, la pente obtenue est liée à la concentration de phénol et il est possible grâce à cette courbe de déterminer la concentration d'un mélange contenant une quantité de phénol inconnue.

### Exemple 4 : Kit à 2 compartiments

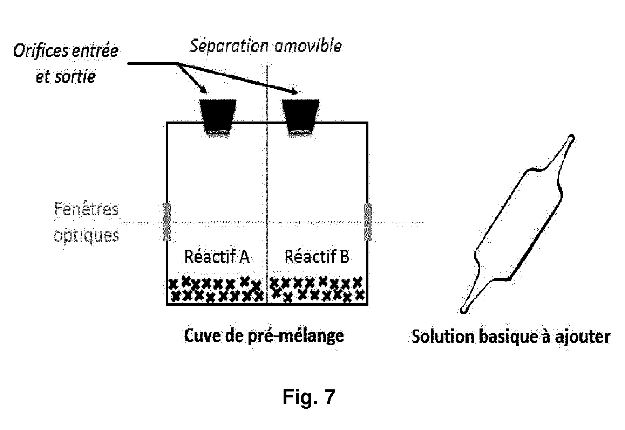

La Figure 7 représente un mode de réalisation d'un kit de l'invention comprenant un récipient clos pourvu de zones (fenêtres optiques) permettant la détection optique par un appareillage approprié, ainsi que d'ouvertures pour la circulation ou l'ajout de liquides ou de gaz.

Le récipient est muni d'une séparation amovible procurant deux compartiments juxtaposés dans lesquels sont installés dans l'un, le réactif capable de générer un produit coloré et dans l'autre le composé oxydant, sous forme solide. Le mélange de ces 2 produits peut être réalisé en retirant la séparation amovible.

On peut ajouter une solution basique préparée à l'avance compte tenu dans un récipient approprié, ici une ampoule, aux constituants solides et mélanger l'ensemble des réactifs, pour obtenir une solution prête à l'emploi.

La détection de phénol ou d'un composé du phénol dans un fluide liquide peut se faire par exemple directement via les ouvertures du récipient. Le barbotage d'un mélange gazeux contenant du phénol est possible directement dans le récipient.

La détection peut se faire directement en reliant un système de détection optique aux fenêtres optiques du récipient.

## Revendications

1. Composition liquide réactive de détection du phénol ou de composés du phénol **caractérisée en ce qu'**elle comprend
- un réactif capable de générer un produit coloré par formation d'une liaison avec le phénol ou un composé du phénol,
- un composé oxydant ou mélange de composés oxydants,
- un composé basique ou mélange de composés basiques,
**en ce que** le ratio [réactif capable de générer un produit coloré] / [composé oxydant] est de 1/2 à 50/1,
et **en ce qu'**elle a un pH supérieur à 7.

2. Composition liquide réactive selon la revendication 1, **caractérisée en ce que** le réactif capable de générer un produit coloré est la 4-aminoantipyrine (AAP) ou un dérivé de l'AAP de formule I 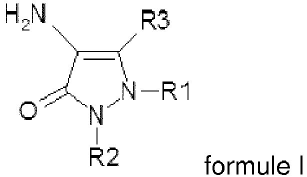 où : R1 est un radical alkyl renfermant de 1 à 30 atomes de carbone, linéaire ou ramifié, ou un radical aryl; R2 est un radical phényl ou para-aminophényl; R3 est un radical alkyle renfermant de 1 à 30 atomes de carbone, linéaire ou ramifié, ou phényl éventuellement substitué.

3. Composition liquide réactive selon la revendication 1 ou 2, **caractérisée en ce que** le composé oxydant ou les composés oxydants sont choisis dans le groupe constitué par le persulfate de potassium, le peroxomonosulfate de potassium ; le peroxyde d'hydrogène et l'hexacyanoferrate de potassium.

4. Composition liquide réactive selon l'une des revendications 1 à 3, **caractérisée en ce que** le composé basique ou mélange de composés basiques comprend une solution tampon choisie dans le groupe constitué par le tampon phosphate, 2-amino-2-(hydroxyméthyl)-1,3-propanediol, carbonate, glycine-soude, ou borate, pour obtenir un pH basique.

5. Composition liquide réactive selon l'une des revendications 1 à 4, **caractérisée en ce que**
- le réactif capable de générer un produit coloré est l'AAP,
- le composé basique ou mélange de composés basiques est choisi parmi un tampon phosphate ou un tampon borate, et
- le composé oxydant ou les composés oxydants est l'hexacyanoferrate de potassium ou un mélange d'hexacyanoferrate de potassium et un ou plusieurs autres composés oxydants.

6. Composition liquide réactive selon l'une des revendications 1 à 5, **caractérisée en ce que** le ratio [réactif capable de générer un produit coloré] / [composé oxydant] est de 1/2 à 10/1.

7. Composition liquide réactive selon l'une des revendications 1 à 6, **caractérisée en ce que** dans la composition liquide réactive de l'invention, les réactifs sont présents en quantités telles que, après mélange, les concentrations en réactif capable de générer un produit coloré sont égales à 1,0.10⁻³ M à 5.10⁻⁵ M.

8. Composition liquide réactive selon l'une des revendications 1 à 7, **caractérisée en ce que** dans la composition liquide réactive de l'invention, les réactifs sont présents en quantités telles que, après mélange, les concentrations en oxydant sont égales à : 2,0.10⁻³ M à 2.10⁻⁶ M.

9. Kit **caractérisé en ce qu'**il comprend séparément
- un réactif capable de générer un produit coloré permettant à une couleur de se développer par formation d'une liaison avec le phénol ou un composé du phénol,
- un composé oxydant ou mélange de composés oxydants,
- un composé basique ou d'un mélange de composés basiques,
où le ratio [réactif coloré] / [composé oxydant] est de 1/2 à 50/1.

10. Kit selon la revendication 9, **caractérisé en ce que** le réactif capable de générer un produit coloré et le composé oxydant sont sous forme solide, et le composé basique ou mélange de composés basiques se trouve en solution.

11. Un procédé en phase liquide d'analyse d'un fluide pouvant contenir du phénol ou un composé du phénol **caractérisé en ce que** l'on met en contact ledit fluide avec une composition liquide réactive telle que définie à l'une des revendications 1 à 8 et **en ce que** l'on observe ou mesure l'éventuelle coloration obtenue.

12. Un procédé pour la dépollution sélective du phénol ou de ses composés, **caractérisé en ce que** l'on met en contact un fluide contenant du phénol ou un de ses composés avec une composition liquide réactive telle que définie à l'une des revendications 1 à 8.
